(19) Europäisches Patentamt
European Patent Office
Office européen des brevets

(11) Publication number : **0 303 357 B1**

(12) **EUROPEAN PATENT SPECIFICATION**

(45) Date of publication of patent specification : **04.03.92 Bulletin 92/10**

(51) Int. Cl.$^5$ : **A61K 31/47**

(21) Application number : **88306378.6**

(22) Date of filing : **13.07.88**

(54) **4-Amino-6,7-dimethoxy-2-(6,7-dimethoxy-1,2,3,4-tetrahydroisoquinol-2-yl)quinoline for (treatment of cardiac arrhythmias).**

(30) Priority : **17.07.87 GB 8716972**

(43) Date of publication of application : **15.02.89 Bulletin 89/07**

(45) Publication of the grant of the patent : **04.03.92 Bulletin 92/10**

(84) Designated Contracting States : **AT BE CH DE FR GB IT LI LU NL SE**

(56) References cited :
**EP-A- 0 100 200**
**BR. J. PHARMACOL., vol. 95, December 1988, pages 1241-1254, The Macmillan Press Ltd.; A.G.C. UPRICHARD et al.: "Effects of the myocardial-selective alpha1-adrenoceptor antagonist UK-52046 and atenolol, alone and in combination, on experimental cardiac arrhythmias in dogs".**

(56) References cited :
**CLIN. SCI., vol. 74, (Suppl. 18), 1988 (Meeting December 1987), page 16, abstract no. 56; N.A. FLORES et al.: "Electrophysiological and anti-arrhythmic effects of UK 52046-27 during ischaemia and reperfusion in the guinea pig heart".**
**CARDIOVASC. Clin., vol. 15, no. 3, 1985, pages 199-248; L.A. SIDDOWAY et al.: "Clinical pharmacology of old and new antiarrhythmic drugs".**

(73) Proprietor : **Pfizer Limited**
**Ramsgate Road**
**Sandwich Kent CT13 9NJ (GB)**

(72) Inventor : **Davey, Michael John, Dr.**
**The Oasthouse Wayhill**
**Minster Ramsgate Kent (GB)**

(74) Representative : **Bradbrook, Geoffrey William**
**PFIZER LIMITED Ramsgate Road**
**Sandwich Kent, CT13 9NJ (GB)**



Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to the use of a particular known quinoline derivative for the manufacture of a medicament for the prevention or reduction of cardiac arrhythmias. It also provides novel pharmaceutical compositions comprising this compound in unit dosage form, or in combination with a beta-adrenoceptor blocking agent, for the treatment of cardiac arrhythmias.

The particular known quinoline derivative of the invention is described and claimed in European Patent no. 0100200 B1 granted on 6th May 1987, being the subject of Example 22 thereof, namely 4-amino-6,7-dimethoxy-2-[6,7-dimethoxy-1,2,3,4-tetrahydroisoquinol-2-yl] quinoline, which has the formula:

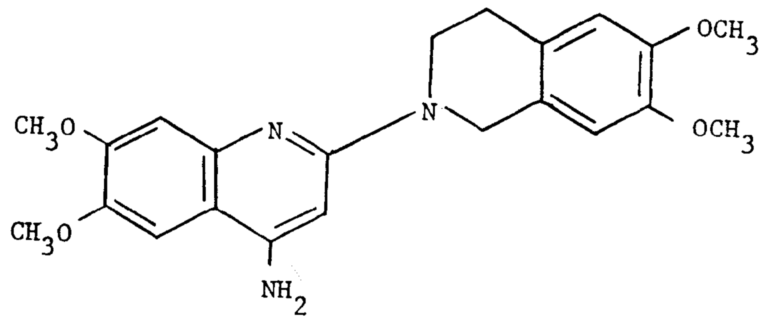

--- (I)

It is also disclosed as Example 34 of the corresponding European patent application no. 0100200 A1 published on 8th February 1984 and United States Patent no. 4,656,174 issued 7th April 1987.

In the above mentioned publications the compound of formula (I) is included among a large number of quinoline derivatives which are described as being useful in therapy as being highly potent antihypertensive agents, the compound of formula (I) being one of the specifically preferred compounds for this purpose. Such compounds are stated to be useful as regulators of the cardiovascular system and, in particular, for the treatment of hypertension. Their antihypertensive activity is stated to be shown by their ability to lower the blood pressure of conscious, spontaneously hypertensive rats and conscious renally hypertensive dogs, when administered orally at doses of up to 5 mg/kg. Administration to humans for the treatment of hypertension can, it is stated, be either by the oral or parenteral routes and oral dosage levels will be at dosage levels within the range 1 to 50 mg/day for an average adult patient (70 kg) and intravenous dose levels will be at 1/5th to 1/10th of the daily oral dose. Thus for an average adult patient individual doses in tablet or capsule form will be approximately in the range from 1 to 25 mg of the active compound.

The preparation of the compound of formula (I) is described in the above mentioned publications and also by Campbell, Hardstone and Palmer in "Tetrahedron Letters", Vol.25, No. 42, pp. 4813-4816 (1984) as entry no. 5 in Table I on page 4814.

It has now surprisingly been discovered that the compound of formula (I) has valuable and unique properties, useful in the treatment of cardiac arrhythmias, not possessed by other compounds disclosed in the above publications, at dose levels much lower than those at which it is effective as an antihypertensive agent.

According to the invention, therefore, there is provided the use of the compound of formula (I), or a pharmaceutically acceptable acid salt thereof, for the manufacture of a medicament for the prevention or reduction of cardiac arrhythmias.

The ability of the compound of formula (I) to reduce or prevent cardiac arrhythmias has been assessed by its antagonism of adrenaline-induced ventricular dysrhythmias when administered intravenously to anaesthetised dogs. The arrhythmic dose of adrenaline (ADA) is defined as that dose which causes at least 3 ventricular ectopic beats within a period of 15 seconds, either during a 3 minute continuous intravenous infusion of adrenaline, or within the 1 minute immediately following cessation of such infusion, to dogs anaesthetised with 1% halothane in oxygen. A single intravenous administration of the compound of formula (I) at dose levels of from 3 to 100 micrograms/kg prior to infusion of adrenaline caused dose-related increases in the ADA. Similarly intravenous or oral administration of the compound of formula (I) to conscious dogs at dose levels of 12.5 micrograms/kg intravenously, or 50 and 200 micrograms/kg orally, respectively 1 hour and 2 hours prior to performing an acute experiment in which the ADA was similarly measured caused marked, long lasting increases in the ADA. No behavioural or symptomological effects were observed following administration of the compound of formula (I) up to the time the dogs were anaesthetised for the acute experiment. At these dose levels, administration of the compound of formula (I) had minimal effects on blood pressure and heart rate.

When the compound of formula (I) was given orally to conscious normotensive dogs at a dose level of 250

micrograms/kg once daily for 4 days it produced a long-lasting significant protection against adrenaline-induced arrhythmias with no significant changes in heart rate or blood pressure. No tolerance or accumulation was observed.

The effect of a dose of 12.5 micrograms/kg of the compound of formula (I) during brief periods of myocardial ischaemia followed by reperfusion in anaesthetised dogs was investigated. The compound prevented the ischaemia-associated increase in total peripheral resistance, caused only a small reduction in the repayment of coronary blood flow debt and tended to maintain stroke volume during coronary artery occlusion. It was without effect on blood pressure and caused only small increases in cardiac output, heart rate, and dP/dt max. (rate of rise of left ventricular pressure), and a very small reduction in filling pressure.

In a conscious dog model of myocardial infarction in which a thrombogenic copper coil was placed in a side branch of the left circumflex coronary artery, an intravenous dose of 25 micrograms/kg of the compound of formula (I) caused a long-lasting suppression of adrenaline-induced ventricular, arrhythmias and suppressed bouts of ischaemia-induced ventricular tachycardia. In a conscious dog model of myocardial infarction, the minimum effective dose of the compound of formula (I) required to reduce adrenaline-induced ventricular arrhythmias was 0.05 micrograms/kg, administered intravenously, and marked suppression occurred after a total intravenous dose of 0.4 micrograms/kg. At these dose levels the compound was without effect on heart rate and arterial pressure and the haemodynamic effects of standing were not modified in any way.

The effects of the compound of formula (I), in intravenous doses from 0.1 to 1000 micrograms/kg, on the haemodynamic consequences of standing in conscious dogs were investigated. The effect on lying blood pressure was minimal throughout the dose range used and no falls in standing arterial pressure were observed for dose amounts below 25 micrograms/kg. At doses of above 1.0 micrograms/kg the normal increase in heart rate on standing was prolonged. The effect of intravenous doses of from 0.1 to 5.0 micrograms/kg on the pressor responses to phenylephrine in conscious dogs was observed. Supine arterial pressure and the heart rate were not affected. At dose levels of 1.0 and 5.0 micrograms/kg the compound caused parallel shifts to the right in the log dose - response curve to phenylephrine were observed.

The ability of the compound of formula (I) to reduce or prevent cardiac arrhythmias has also been assessed by its ability to antagonise picrotoxin-induced ventricular arrhythmias in anaesthetised cats. Cats were anaesthetised by inhalation of a mixture of halothane, nitrous oxide and oxygen (3:1 v/v), maintained by intravenous injection of chloralose. The clonic convulsions induced by picrotoxin were prevented by an intravenous dose of 0.25mg/kg decamethonium, followed by continuous infusion of 0.5mg/kg/h, and a single dose of 4 mg/kg picrotoxin was then administered intravenously. The compound of formula (I) was effective in promptly restoring normal sinus rhythm when administered at doses of 25 or 50 micrograms/kg 1 to 10 minutes after administration of picrotoxin.

When the compound of formula (I) was administered intravenously to anaesthetised cats at effective anti-arrhythmic dose levels up to 25 micrograms/kg it caused no major changes of arterial pressure, venous pressure, left ventricular pressure, left ventricular end-diastolic pressure, heart rate or maximum or minimum dP/dt. These cardiovascular parameters were maintained at physiological levels even when the cumulative dose level was increased to 16 milligrams/kg. In an anaesthetised cat ischaemia model involving left anterior coronary artery occlusion and sympathetic stimulation, which attempts to combine myocardial ischaemia and sympathetic stimulation, an intravenous dose of 25 micrograms/kg of the compound of formula (I) attenuated malignant ventricular ectopic activity.

On the basis of these experiments, it is expected that for human use in the prevention or reduction of cardiac arrhythmias single, oral dosages of the compound of formula (I) will be in the range from 0.05 to 1.0 mg per day, for example 0.05 to 0.5 mg per day for an average adult patient (70 kg), taken in up to 4 doses per day. Thus individual tablets or capsules might contain between 0.025 and 1.0 mg, for example 0.025 to 0.5 mg, of active compound, in a suitable pharmaceutically acceptable vehicle or carrier. Single dosages for parenteral, e.g. intravenous administration would be expected to be between 0.1 and 1 microgram/kg, such as 0.1 to 0.5, e.g. 5 to 100 micrograms. A severe cardiac arrhythmia is preferably treated by the intravenous route in order to effect a rapid conversion to normal sinus rhythm. Variations on these dosages may occur depending on the weight and condition of the subject being treated, as will be determined by the medical practitioner.

The compound of the formula (I), or a pharmaceutically acceptable acid addition salt thereof, can be administered alone, but will generally be administered in admixture with a pharmaceutical carrier selected with regard to the intended route of administration and standard pharmaceutical practice. They can be administered both to patients suffering from arrhythmias and also prophylactically to those likely to develop arrhythmias. For example they may be administered orally in the form of tablets containing such excipients as starch or lactose, or in capsule either alone or in admixture with excipients, or in form of elixirs or suspensions containing flavouring or colouring agents. They may be injected parenterally, for example, intravenously, intramuscularly or subcutaneously. For parenteral administration, they are best used in the form of a sterile aqueous solution which

may contain other solutes, for example, enough salts or glucose to make the solution isotonic with blood.

The pharmaceutically acceptable acid addition salts of the compound of the formula (I) are salts formed from acids which form non-toxic salts such as the hydrochloride, hydrobromide, hydroiodide, sulphate or bisulphate, phosphate or hydrogen phosphate, acetate, maleate, fumarate, lactate, tartrate, citrate, gluconate, benzoate, methanesulphonate, benzenesulphonate and p-toluenesulphonate salts.

It has also been discovered that the antiarrhythmic properties of the compound of formula (I) are enhanced by use in combination with a non-selective beta-adrenoceptor blocking compound, such as propranolol (1-(isopropylamino)-3-(1-naphthyloxy)-2-propanol), or with a cardio-selective beta-adrenoceptor blocking agent, such as atenolol (2-[p-[2-hydroxy-3-(isopropylamino)propoxy]phenyl]acetamide).

In similar experiments to those already described above, the increase in the ADA in anaesthetised dogs caused by intravenous administration of 100 micrograms/kg of the compound of formula (I) together with 1 mg/kg of propranolol or 1 mg/kg of atenolol appeared to be more than additive and possibly synergistic. The increases in ADA produced by the combinations were found to be more than twice as great as that of 100 micrograms/kg of the compound of formula (I) alone, and more than five times and three times respectively as great as that of 1 mg/kg of propranolol alone and that of 1 mg/kg of atenolol alone.

Similarly intravenous administration of 0.36 microgram/kg of the compound of formula (I) together with 0.36 microgram/kg of atenolol was found to be sufficient to prevent ventricular arrhythmias caused by adrenaline in halothane-anaesthetised dogs whereas dosages of about 3.8 microgram/kg and 14.6 microgram/kg were necessary when the compound of formula (I) or atenolol, respectively, were administered alone.

Also according to the invention, therefore, there are provided pharmaceutical compositions in unit dosage form for oral or parenteral administration in the treatment of cardiac arrhythmias comprising form 0.025 to 1.0 mg, or 5 to 100 micrograms, respectively, of the compound of formula (I) or equivalent amounts of a pharmaceutically acceptable acid addition salt thereof, and a beta-andrenoceptor blocking agent, per unit dose.

In the above-described experiments the compound of formula (I) was administered as the methane sulphonate or hydrochloride salt. It has been found that all the salts tested are pharmaceutically equivalent, the quantity of salt used being adjusted to contain the equivalent amount of compound of formula (I).

## Claims

1. Use of the compound of formula (I) or a pharmaceutically acceptable salt thereof:

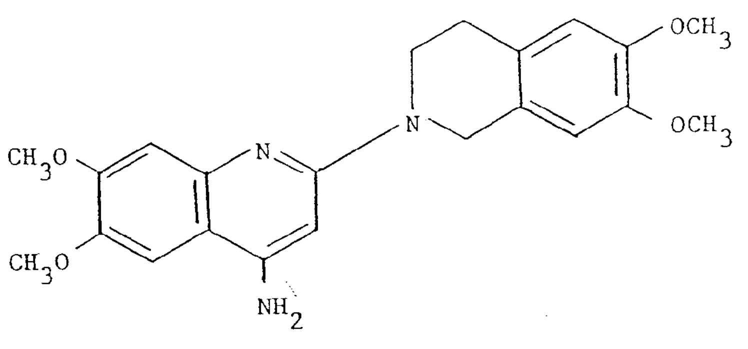

--- (I)

for manufacture of a medicament for the prevention or reduction of cardiac arrhythmias.

2. Use of a combination of the compound of formula (I) as claimed in claim 1 or a pharmaceutically acceptable salt thereof with a beta-adrenoceptor blocking agent for manufacture of a medicament for the prevention or reduction of cardiac arrhythmias.

3. Use according to claim 2, wherein said beta-adrenoceptor blocking agent is propranolol or atenolol.

4. A pharmaceutical composition for oral administration in unit dosage form for preventing or reducing cardiac arrhythmias comprising from 0.025 mg to 1.0 mg of the compound of formula (I) as defined in claim 1, or an equivalent amount of a pharmaceutically acceptable salt thereof, and a beta-adrenoceptor blocking agent, per unit dose.

5. A pharmaceutical composition for parenteral administration in unit dosage form for preventing or reducing cardiac arrhythmias comprising from 5 to 100 micrograms of the compound formula (I) as defined in claim 1, or an equivalent amount of a pharmaceutically acceptable salt thereof, and a beta-andrenoceptor blocking agent, per unit dose.

6. A composition according to claim 4 or 5, in which the beta-adrenoceptor blocking agent is propranolol

or atenolol.

**Patentansprüche**

1. Verwendung der Verbindung der Formel (I) oder eines pharmazeutisch annehmbaren Salzes derselben:

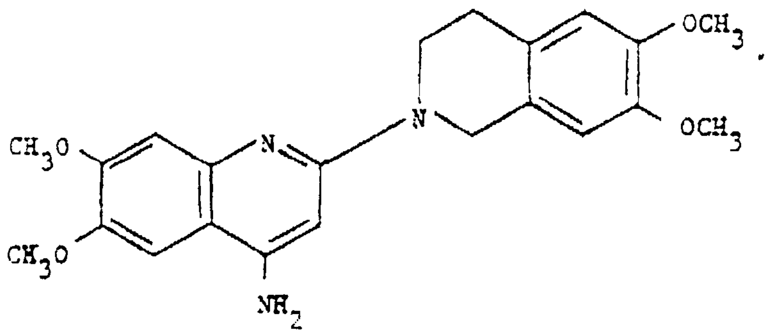

--- (I)

zur Herstellung eines Arzneimittels für die Verhütung oder Verminderung kardialer Arrhythmien.

2. Verwendung einer Kombination der Verbindung der Formel (I) gemäß Anspruch 1 oder eines pharmazeutisch annehmbaren Salzes derselben mit einem ß-Adrenozeptor-Blockermittel zur Herstellung eines Arznemittels für die Verhütung oder Verminderung Kardialer Arrhythmien.

3. Verwendung gemäß Anspruch 2, bei der das ß-Adrenozeptor-Blockermittel Propanolol oder Atenolol ist.

4. Pharmazeutische Zusammensetzung zur oralen Verabreichung in Einheitsdosisform für die Verhütung oder Verminderung kardialer Arrhythmien, die 0,025 bis 1,0 mg der Verbindung der Formel (I) gemäß Definition in Anspruch 1 oder eine äquivalente Menge eines pharmazeutisch annehmbaren Salzes derselben und ein β-Adrenozeptor-Blockermittel pro Dosiseinheit umfaßt.

5. Pharmazeutische Zusammensetzunbg zur parenteralen Verabreichung in Eineitsdosisform für die Verhütung oder Verminderung kardialer Arrhydhmien, die 5 bis 100 μg der Verbindung der Formel (I) gemäß Definition in Anspruch 1 oder eine äquivalente Menge eines pharmazeutisch annehmbaren Salzes derselben und ein α-Adrenozeptor-Blockermittel pro Dosiseinheit umfaßt.

6. Zusammensetzung gemäß Anspruch 4 oder 5, in welcher das α-Adrenozeptor-Blockermittel Propranolol oder Atenolol ist.

**Revendications**

1. Utilisation d'un composé de formule (I) ou d'un sel pharmaceutiquement acceptable d'un tel composé :

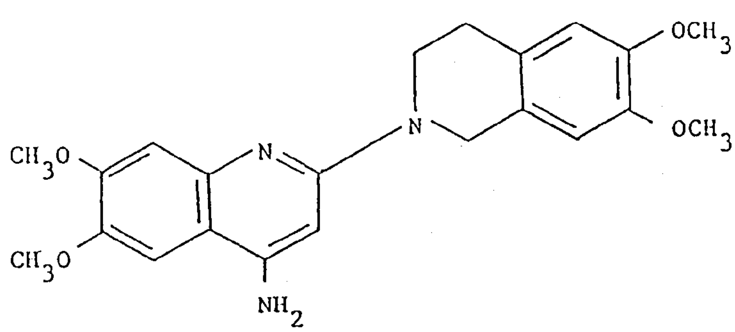

---(I)

pour la fabrication d'un médicament destiné à la prévention ou à la réduction des arythmies cardiaques.

2. Utilisation d'une combinaison du composé de formule (I) selon la revendication 1, ou d'un sel pharmaceutiquement acceptable d'un tel composé, avec un agent bloquant bêta-adrénocepteur pour la fabrication d'un médicament pour la prévention ou la réduction des arythmies cardiaques.

3. Utilisation selon la revendication 2, dans laquelle ledit agent bloquant bêta-adrénocepteur est le propranolol ou l'aténolol.

4. Composition pharmaceutique pour l'administration orale sous forme d'unité dosée pour la prévention ou la réduction des arythmies cardiaques renfermant, par unité dosée, de 0,025 mg à 1,0 mg du composé de formule (I) selon la revendication 1, ou d'une quantité équivalente d'un sel pharmaceutiquement acceptable d'un tel composé, et un agent bloquant bêta-adrénocepteur.

5. Composition pharmaceutique pour l'administration parentérale sous forme d'unité dosée pour la prévention ou la réduction des arythmies cardiaques renfermant, par unité dosée, de 5 à 100 microgrammes du composé de formule (I) selon la revendication 1, ou d'une quantité équivalente d'un sel pharmaceutiquement acceptable d'un tel composé, par unité dosée, et un agent bloquant bêta-adrénocepteur.

6. Composition selon la revendication 4 ou 5, dans laquelle l'agent bloquant bêta-adrénocepteur est le propranolol ou l'aténolol.